# EUROPÄISCHE PATENTSCHRIFT

(11) **EP 1 508 301 B1**
(45) Veröffentlichungstag und Bekanntmachung des Hinweises auf die Patenterteilung: **09.05.2012**
(21) Anmeldenummer: 04019702.2
(22) Anmeldetag: 19.08.2004
(51) Int. Cl.: A61B 5/103, A61N 5/06

(54) **Vorrichtung zur gezielten Bestrahlung des menschlichen Körpers**
Apparatus for controlled irradiation of the human body
Dispositif pour l'irradiation controlée d'un corps humain

(30) Priorität: 19.08.2003 DE 10338004
(43) Veröffentlichungstag der Anmeldung: 23.02.2005
(73) Patentinhaber: JK-Holding GmbH, 53578 Windhagen (DE)
(72) Erfinder: Kratz, Walter, 53578 Windhagen (DE)
(74) Vertreter: Koepe, Gerd L.

(56) Entgegenhaltungen:
- WO-A-92/17242
- WO-A-93/16635
- DE-A- 3 137 326
- DE-A1- 3 533 789
- DE-A1- 4 312 547
- DE-A1- 10 233 984
- DE-A1- 19 810 201
- DE-C- 3 931 036
- DE-U1- 9 406 187
- US-A- 4 882 598
- US-A- 5 636 637
- US-A- 6 070 092
- US-B1- 6 348 694

## Beschreibung

Die vorliegende Erfindung betrifft eine Vorrichtung zur gezielten Bestrahlung des menschlichen Körpers. Insbesondere betrifft die Erfindung eine Vorrichtung zur Bestrahlung des menschlichen Körpers mit bräunender, insbesondere mit einer einen gewissen Anteil an UV-Strahlung enthaltender Strahlung, die der Tatsache Rechnung tragen, daß verschiedene Menschen unterschiedliche Hauttypen und gegen bräunende Strahlung unterschiedlich empfindliche Haut haben und gegebenenfalls auch in unterschiedlichem Maße gewohnt sind, bräunender Strahlung, insbesondere Strahlung mit einem gewissen Anteil an UV-Strahlung, ausgesetzt zu sein.

Bräunungsgeräte auf der Basis von UV-Strahlem sind in unterschiedlichen Ausbildungen und Größen bekannt. Besonderer Beliebtheit erfreuen sich Bräunungsgeräte zur Ganzkörper-Bräunung. Diese weisen als Unterteil eine gegebenenfalls mit einem Profil versehene, Liege ("Bank") auf, die unterhalb einer für die bräunende Strahlung transparenten Oberfläche mit die bräunende Strahlung abstrahlenden UV-Strahler-Röhren bestückt ist. Dem Unterteil der Bräunungsgeräte entspricht ein bewegliches Oberteil, das entweder um eine parallel zu einer Kante, üblicherweise parallel zu einer der beiden längeren Kanten, der Liege verlaufende Achse schwenkbar oder an einem Stativ oder an einer Aufhängung an einer Raumdecke in vertikaler Richtung bewegbar ist. Das Oberteil ist - oberhalb einer für die bräunende Strahlung transparenten unteren Fläche - ebenfalls mit die bräunende Strahlung abstrahlenden UV-Strahler-Röhren bestückt. Benutzer eines Bräunungsgerätes heben oder schwenken das Oberteil in die offene Position, die ein bequemes "Einsteigen" ermöglicht, legen sich auf die Liege, schließen das Oberteil und schalten dann die UV-Strahler-Röhren für eine vorgewählte Bräunungszeit ein, die im Bereich weniger Minuten, jedoch durchaus auch bei 15 Minuten oder länger liegen kann. Ein Bräunungsverfahren unter Verwendung der vorgenannten, oben nur hinsichtlich wichtiger allgemeiner Merkmale angesprochenen Vorrichtung ist ebenfalls bekannt und damit Stand der Technik.

Derartige Bräunungsgeräte finden in zunehmendem Maße Verwendung, und zwar sowohl im kosmetischen Bereich (sowohl private Anwendung daheim als auch in sogenannten "Sonnenstudios", die professionell genutzte Geräte zum Bräunen der Haut bereitstellen), als auch im medizinischen Bereich, in dem die präventive oder therapeutische Behandlung der Haut mehr und mehr als medikamentenfreier oder die medikamentöse Behandlung begleitender Behandlungsweg erfolgreich eingesetzt wird (Akne, Psoriasis usw.).

Grundsätzlich sind im Stand der Technik verwendete Bräunungsgeräte für alle vorgenannten Zwecke so ausgelegt, daß ein Bräunungsvorgang stattfinden kann, ohne daß die Haut durch Komponenten der durch die verwendeten Röhren abgestrahlten Strahlung, insbesondere durch UV-Strahlungskomponenten, geschädigt wird, solange Gewohnheiten und Schutzmaßnahmen eingehalten werden, die die sich dem Bräunungsvorgang unterziehende Person auch bei einer Exposition natürlicher Sonnenstrahlung beachten muß. Mit anderen Worten: Genauso wie beim Bräunen in der Natur muß eine Person mit "heller" Haut und/oder wenig vorhergehenden Gelegenheiten, der Sonnenstrahlung und/oder künstlicher UV-Strahlung (z. B. eines Bräunungsgeräts) ausgesetzt zu sein, geringere Bestrahlungdosen und/oder kürzere Bestrahlungszeiten wählen und/oder die Haut stärker mit üblichen (z. B. kosmetischen) Mitteln schützen als eine Person mit "dunkler" Haut und/oder mehr vorangehenden Gelegenheiten, der Sonnenstrahlung und/oder künstlicher UV-Strahlung ausgesetzt zu sein.

Im medizinischen Bereich kann die Bestrahlungsdosis und/oder Bestrahlungszeit empirisch, in jedem Fall jedoch unter Aufsicht einer die Bestrahlungsdosis und/oder Zeit steuernden Person (z. B. eines Arztes oder einer von ihm instruierten Person), gesteuert werden. Üblicherweise wird bei medizinischer Strahlenbehandlung zu Beginn der Bestrahlungstherapie eine geringe konstante Strahlungsdosis und/oder kurze Behandlungszeit gewählt, die Reaktion der Haut der bestrahlten Person auf die Bestrahlung ermittelt und die Dosis bzw. Zeit für folgende Schritte der Strahlenbehandlung entsprechend der Erstreaktion eingestellt, üblicherweise langsam gesteigert. Dies erfordert eine gewisse Erfahrung, d. h. sowohl Erfahrung mit der Behandlungsmethode im allgemeinen als auch in der Einschätzung der physiologisch erzielten Reaktion als auch im Umgang mit dem verwendeten Bräunungsgerät. Unterschiedlichen Reaktionen und Hauttypen kann nur über eine Reduktion der (üblicherweise konstanten) Strahlungsdosis oder eine Kürzung der Bestrahlungszeit Rechnung getragen werden.

Im kosmetischen Bereich stehen bei der professionellen Bereitstellung von Bräunungsgeräten zwar üblicherweise Personen bereit, die eine Beratung hinsichtlich Bräunungszeit und Strahlungsdosis geben können, doch wird diese Beratung häufig nicht angenommen. Im häuslichen Bereich fehlt eine solche Beratung ganz. Vielmehr wählt die ein Bräunungsgerät benutzende Person meist die Bräunungszeit und Bestrahlungsdosis am Gerät selbst, so daß selbst dann, wenn vorher eine fachkundige Beratung erfolgt ist, dies nicht sicherstellt, daß nicht eine stärkere Dosis oder längere Bräunungszeit gewählt wird, beispielsweise in dem irrigen Bestreben, so den Vorgang der Bräunung beschleunigen oder in einer "Sitzung" eine tiefere Hautbräunung erzielen zu können. Dies führt - bekanntermaßen wie in der Natur - zu durch die UV-Strahlung an der Haut erzeugten Erythemen, landläufig bekannt als "Sonnenbrand", die sehr schmerzhaft sein können und auf Dauer die Haut auch schädigen.

Die Druckschrift DE-A 43 12 547 beschreibt bereits Bräunungsgeräte, bei denen die Risiken einer zu starken Bestrahlung der Haut sowohl im medizinischen Bereich als auch im kosmetischen Bereich deutlich verringert werden können. Dies geschieht gemäß der genannten Druckschrift mittels einer Anpassung der UV-Strahlung eines Bräunungsräts an den individuellen Hauttyp des jeweiligen Benutzers. Die UV-Strahler ("Röhren) werden jeweils mit einer separaten Einrichtung zur Leistungsregelung versehen, und mehrere dieser Einrichtungen zur Leistungsregelung sind mit einer zentralen Steuereinrichtung verbunden, so daß eine individuelle Regelung einzelner Röhren erfolgen kann. In der Praxis erfolgt die Leistungsregelung über eine (an sich seit langer Zeit bekannte) Vorrichtung zur Ermittlung der individuellen Hautfärbung der zu bestrahlenden Person. Direkt auf der Haut der zu bestrahlenden Person wird über einen Abtastkopf optisch der Hauttyp bzw. die Hautfärbung der zu bestrahlenden Person ermittelt. Dies geschieht durch Einstrahlen von Licht einer definierten spektralen Zusammensetzung, Abtasten des von der Haut reflektierten Lichts und Ermitteln des Verhältnisses des absorbierten/reflektierten Lichts zum eingestrahlten Licht. Die zentrale Steuereinrichtung wird mit den ermittelten (und mit empirischen Daten abgeglichenen) Daten gespeist und bestimmt die maximale Strahlungsleistung, die die zu bestrahlende Person empfangen darf, und - je nach der gewünschten Länge der Bestrahlung - auch die im individuellen Fall zur Anwendung kommende maximale Bestrahlungszeit.

Nachteilig an dem genannten Vorschlag ist, daß der Tatsache nicht Rechnung getragen wird, daß unterschiedliche Teile jedes menschlichen Körpers unterschiedlich empfänglich für bräunende Strahlung und auch unterschiedlich empfindlich gegenüber bräunender Strahlung, insbesondere UV-Strahlung, sind. So ist der Körper (und sind insbesondere Partien des Körpers, die üblicherweise bekleidet sind) wesentlich empfindlicher gegenüber UV-Strahlung als das Gesicht. Messungen der Hautbräunung bzw. des Hauttyps am Gesicht führen also regelmäßig zu einer höheren, maximal erlaubten Strahlungsdosis gemäß der in der Druckschrift DE-A 43 12 547 beschriebenen Vorrichtung als Messungen der Hautbräunung bzw. des Hauttyps am Körper, beispielsweise an der Brust oder an der Bauchdecke. Die Folge solcher "Fehlmessungen", obwohl für den gemessenen Hauttyp an der bestimmten Stelle der Hautoberfläche korrekt, sind zu hohe oder zu niedrige Strahlungsdosen an anderen Stellen der Hautoberfläche derselben Person. Insbesondere für den gewählten Fall der Vorab-Messung des Hauttyps bzw. der Hautbräunung an einer der regelmäßig weniger empfindlichen Stellen (z. B. am Gesicht der zu bestrahlenden Person) wird der Körper (und werden insbesondere empfindliche Stellen des Körpers) mit einer zu starken Dosis an bräunender Strahlung bestrahlt, was zu der bekannten erythemalen Reizung der Haut oder sogar zu Schädigungen der empfindlichen Haut führt.

Die Erfindung schafft Abhilfe für diesen als nachteilig empfundenen Umstand des Standes der Technik. Überraschenderweise wurde nämlich gefunden, daß es bei Durchführung mehrerer Messungen des Hauttyps bzw. des Status der Hautbräunung einer zu bestrahlenden Person an verschiedenen Stellen der Hautoberfläche und separater Einstellung der auf die jeweilige Körperpartie auftreffenden Strahlung entsprechend den Ergebnissen der jeweiligen Messung durch separate Regelung der die Strahlung abgebenden UV-Strahlungsquellen wie beispielsweise UV-Strahler-Röhren möglich ist, Überbestrahlungen zu vermeiden und die für eine bestimmte Hautpartie zulässige maximale Strahlungsdosis optimal auszunutzen.

Das Dokument US-A 5,636,637 betrifft ein Verfahren (und eine Vorrichtung) zur Bestimmung von Kenngrößen, die charakteristisch für das Verhalten einer Lichtstrahlung ausgesetzten Oberfläche und insbesondere der Haut eines Menschen sind.

Die Druckschrift US-B 6,348,694 betrifft ein Verfahren und ein System zum Testen, welche Sonnenschutzmittel welche Schutzwirkung gegen UV-Strahlung auf der menschlichen Haut entfalten. Dazu wird in einer in Figur 2 von (D2) gezeigten Vorrichtung Umweltstrahlung (100) in zwei getrennten Strahlenkanälen (30) und (40) als Strahlung (36, 38) auf die mit Sonnenschutzmittel (26) beaufschlagte menschliche Haut (24) einerseits und auf einen Referenzreflektor (50) geleitet. Die von der Haut mit dem Sonnenschutzmittel reflektierte Strahlung wird auf ein Paar Sensoren (32, 34) reflektiert, während die von dem Referenzreflektor (59) reflektierte Strahlung auf ein Paar Sensoren (42, 44) reflektiert wird.

Die vorliegende Erfindung betrifft demgegenüber eine Einrichtung zur Messung des für die Bemessung einer UV-Bestrahlungsdosis relevanten Zustands der Haut eines Menschen auf dessen Haut-Oberfläche nach Anspruch 1.

Die Erfindung betrifft weiter die Verwendung einer Einrichtung nach der nachfolgenden detaillierten Beschreibung in einer wenigstens eine Einrichtung mit einer dem Benutzer zugewandten, für die Strahlung transparenten Fläche und mehreren, auf der vom Benutzer entfernten Seite der Fläche angeordneten Gruppen von UV-Strahlungsquellen, die hinsichtlich Zahl und Anordnung so beschaffen sind, daß sie die einen UV-Anteil umfassende Strahlung im wesentlichen getrennt auf verschiedene Teile des Körpers des Benutzers richten können, und die steuerbar sind durch eine oder mehrere Einrichtung(en) zur Regelung der Leistung der UV-Strahlungsquellen, umfassenden Vorrichtung zur Bestrahlung des Körpers eines Benutzers mit einen UV-Strahlungsanteil umfassender Strahlung zur Regelung der Leistung nach Ergebnissen von Messungen des für die Bemessung der UV-Bestrahlungsdosis relevanten Zustands der Haut des Benutzers an mehreren Stellen seiner Haut-Oberfläche.

Die Erfindung betrifft auch die Verwendung der obigen Einrichtung zur Messung des für eine Bemessung einer UV-Bestrahlungsdosis relevanten Zustandes der Haut eines Menschen im Zusammenhang mit der Bestrahlung der Haut mit einen UV-Strahlungsanteil umfassender Strahlung in einem Bräunungsgerät mit künstlichen UV-Strahlungsquellen oder zur Messung des für eine Bemessung einer UV-Bestrahlungsdosis relevanten Zustandes der Haut eines Menschen im Zusammenhang mit der Bestrahlung der Haut mit einen UV-Strahlungsanteil umfassender Strahlung in der Natur oder zur Messung des für eine Bemessung einer UV-Bestrahlungsdosis relevanten Zustandes der Haut eines Menschen im Zusammenhang mit der medizinischen Bestrahlung der Haut mit einen UV-Strahlungsanteil umfassender Strahlung oder zur Messung des für eine Bemessung einer UV-Bestrahlungsdosis relevanten Zustandes der Haut eines Menschen im Zusammenhang mit der kosmetischen Bestrahlung der Haut mit einen UV-Strahlungsanteil umfassender Strahlung.

Schließlich betrifft die Erfindung auch ein Verfahren zur Ermittlung des für eine Bemessung einer UV-Bestrahlungsdosis relevanten Zustand der Haut des Benutzers eines Bräunungsgeräts, in dem man die Absorption und/oder Reflexion von auf die Haut eingestrahlter Strahlung definierter spektraler Zusammensetzung im Verhältnis zur eingestrahlten Strahlung an mehreren Stellen seiner Haut-Oberfläche mittels einer oder mehrerer Einrichtung(en) 200 gemäß der nachfolgenden detaillierten Beschreibung mißt und entsprechend den Ergebnissen der Messungen die Leistung einzelner Gruppen der UV-Strahler-Röhren des Bräunungsgeräts regelt, die ihre Strahlung im wesentlichen getrennt auf verschiedene Teile des Körpers des Benutzers richten können.

Die Erfindung wird weiter anhand der in den Figuren gezeigten bevorzugten Ausführungsformen erläutert. Die in den Figuren gezeigten Ausführungsformen dienen der Erläuterung der Erfindung, nicht deren Beschränkung. Es zeigen:
Figur 1 eine Einrichtung zur Messung des für die Bemessung einer UV-Strahlungsdosis relevanten Zustands der Haut, insbesondere der Hautfarbe und/oder des Haut-Bräunungszustandes, eines Menschen in perspektivischer Ansicht;
Figur 2 eine Einrichtung gemäß Figur 1 in einer Explosionsdarstellung;
Figur 3 eine Einrichtung gemäß Figur 1 in Vorderansicht (Figur 3A), in seitlicher Ansicht (Figur 3B) und in Aufsicht (Figur 3C) sowie im seitlichen Schnitt (Figur 3D) und im Längsschnitt (Figur 3E);
Figur 4 eine seitliche Schnittansicht des Funktionsbereichs der Einrichtung gemäß Figur 1;
Figur 5 eine Vorrichtung zur Bestrahlung des menschlichen Körpers mit einer einen UV-Strahlungsanteil umfassenden Strahlung unter besonderer Berücksichtigung der Einrichtung zur Messung der Hautfarbe und/oder des Haut-Bräunungszustandes;

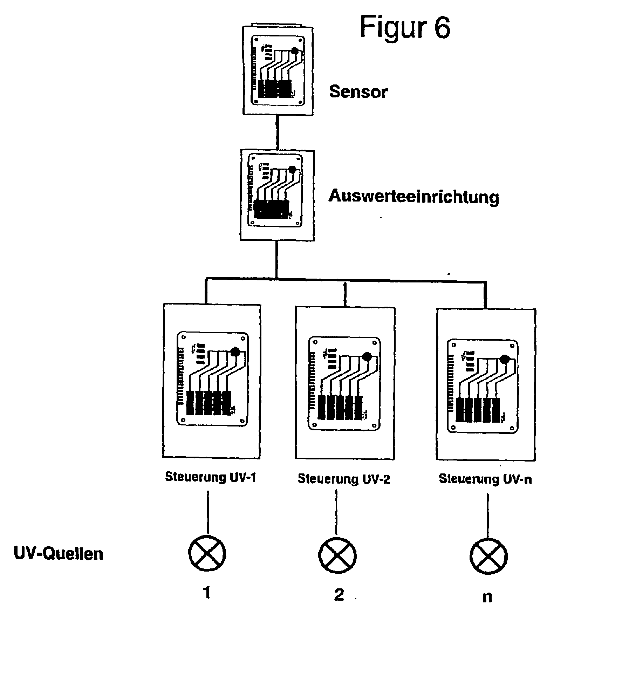

Figur 6 ein Schaltbild, das die Ansteuerung von UV-Strahlungsquellen und insbesondere UV-Strahler-Röhren durch die Auswertung der Ergebnisse der Messung der Hautfarben-Meßeinrichtung zeigt; und
Figur 7 ein Fließdiagramm, das den Ablauf des Verfahrens zur Bestrahlung des menschlichen Körpers unter vorheriger und/oder gleichzeitiger Ermittlung der Hautfarbe und/oder des Haut-Bräunungszustandes zeigt.

Die Erfindung wird nun unter Bezugnahme auf die Figuren im einzelnen beschrieben.

Der Begriff "Hautfarbe" wird im Rahmen der Erfindung verstanden als Summe der Wellenlängen des reflektierten Lichts, das infolge der Einstrahlung von Licht einer definierten Intensität und Wellenlänge von der Hautoberfläche reflektiert wird. Die Hautfarbe dient als eine Grundlage für die Beurteilung der Frage, welche Dosis an ultravioletter Strahlung die Haut vertragen kann, ohne auch nur im Ansatz erythemal gereizt zu werden. Die Haut unterschiedlicher Individuen reagiert - wie gefunden wurde - unterschiedlich auf auf die Hautoberfläche eingestrahltes Licht einer definierten spektralen Zusammensetzung für die Messung eines für die Bemessung der UV-Strahlungsdosis relevanten Zustandes der Haut. Haut von Menschen mit heller Hautfarbe reagiert üblicherweise deutlich empfindlicher (und verträgt daher bis zum Erreichen einer erythemalen Reizung deutlich weniger UV-Strahlung) als Haut von Menschen mit einer dunklen Hautfarbe. Das von der Oberfläche der Haut einer speziellen Hautfarbe reflektierte Licht liefert ein gutes Maß dafür, wie empfindlich die jeweilige Haut für UV-Strahlung ist, wie sie für den Vorgang des Bräunens der Haut verwendet wird.

Im Unterschied zu dem Begriff "Hautfarbe" wird im Rahmen der vorliegenden Erfindung unter dem Begriff "Bräunungszustand der Haut" der bereits als Reaktion auf die Bestrahlung der Haut mit UV-Strahlung erreichte, durch Pigmentierung hervorgerufene Zustand der Haut zum Meßzeitpunkt verstanden. Erfahrungsgemäß reagiert die pigmentierte ("dunkle") Haut weniger empfindlich als wenig pigmentierte ("helle") oder sogar unpigmenterte Haut auf UV-Strahlung. Unabhängig von der Hautfarbe liefert also der Haut-Bräunungszustand ein weiteres gutes Maß dafür, welche Dosis von UV-Strahlung die Haut eines Menschen empfangen kann, ohne erythemal gereizt zu werden.

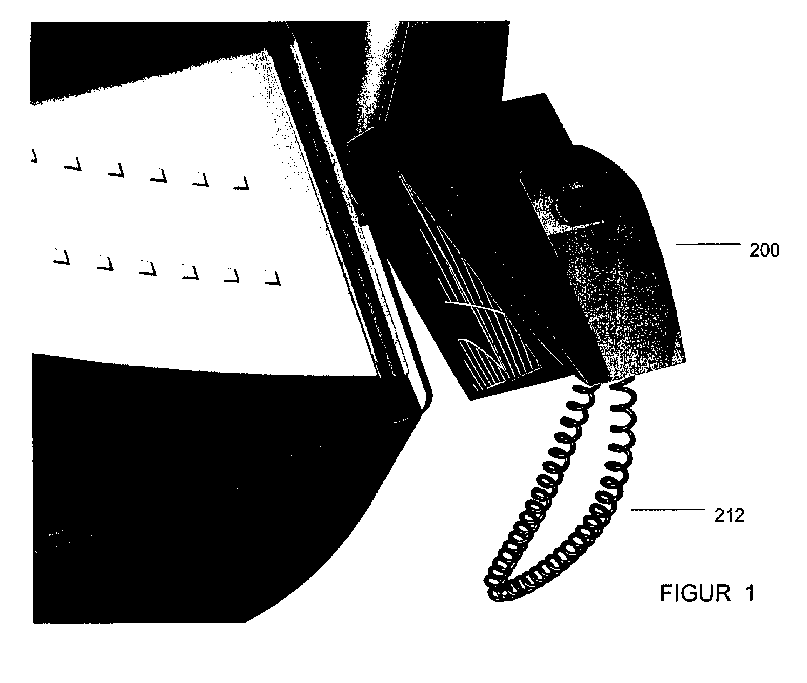

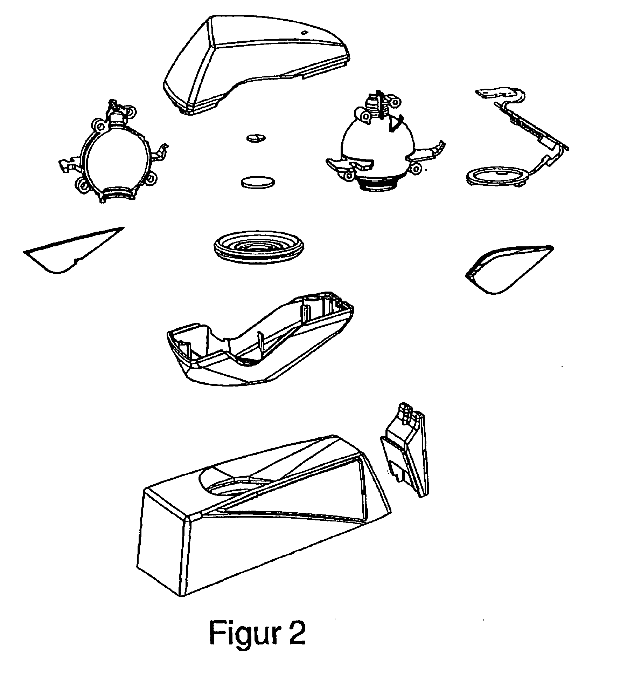

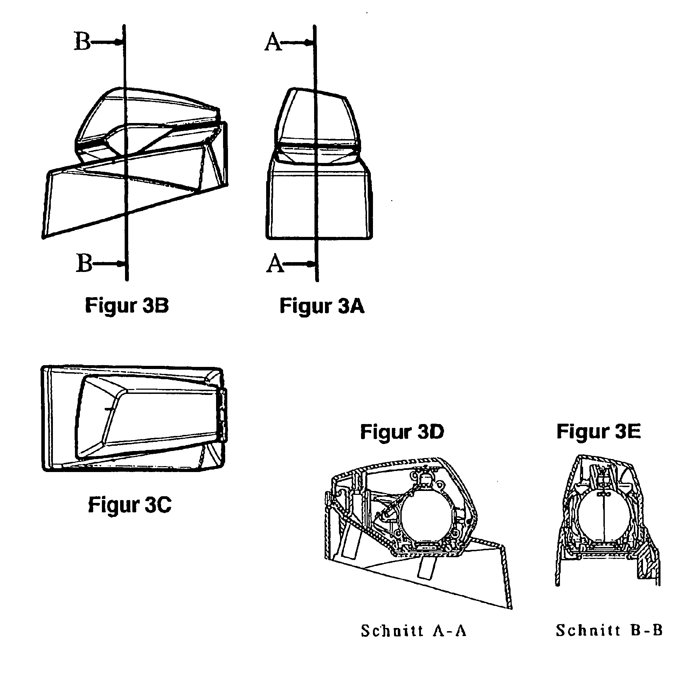

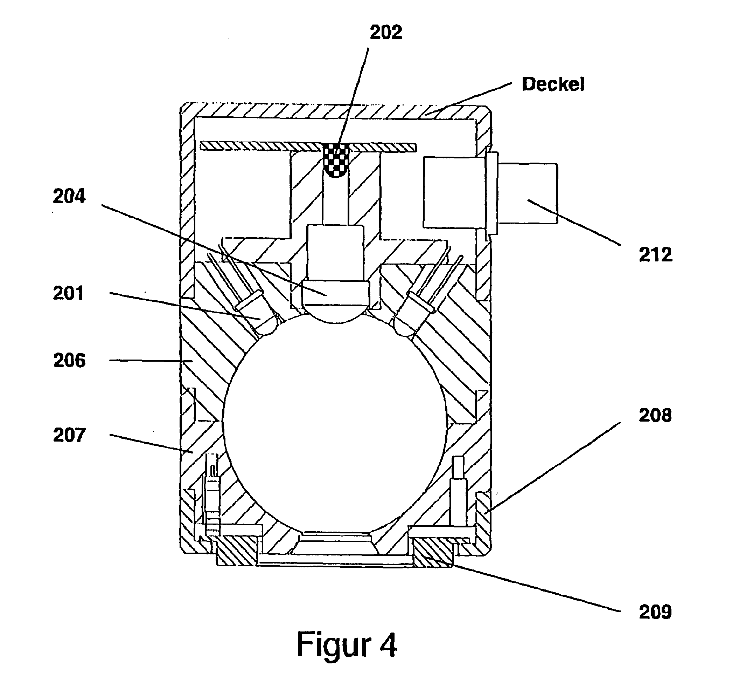

Die erfindungsgemäße Einrichtung 200 zur Messung des für eine Bemessung einer UV-Bestrahlungsdosis relevanten Zustandes der Haut eines Menschen und - besonders bevorzugt - zur Messung der Hautfarbe und/oder des Haut-Bräunungszustandes eines Menschen ist in den Figuren 1 bis 4 gezeigt. Allgemein erfolgt die Messung der Hautfarbe eines Menschen und/oder die Messung des Haut-Bräunungszustandes eines Menschen mittels einer Einrichtung 200, die umfaßt: (a) eine Einrichtung 201 zur Abgabe bzw. Emission von Strahlung definierter Zusammensetzung auf die Haut-Oberfläche; (b) gegebenenfalls eine Einrichtung 211 zum Heranführen der Strahlung definierter Zusammensetzung an die Emissionseinrichtung 201; (c) einen Sensor 202 zur Messung von von der Hautoberfläche reflektierter Anteile der von der Emissionseinrichtung 201 eingestrahlten Strahlung; sowie (d) Einrichtungen 212 zur Versorgung der Einrichtung 201 und/oder des Sensors 202 mit Energie und zur Übertragung von Meßdaten an eine Auswert-Einrichtung 203.

In bevorzugten Ausführungsformen der Erfindung kann es sich bei der Einrichtung 201 zur Abgabe bzw. Emission von Strahlung definierter Zusammensetzung um eine oder mehrere Licht emittierende Dioden (LED's) oder um einen Lichtleiter handeln. Erstere können einzeln oder in Gruppen von mehreren LED's verwendet werden; diese können (müssen jedoch nicht zwingend) hinsichtlich der Wellenlänge des emittierten Lichts unterschiedlich sein. Ein Lichtleiter führt Licht definierter spektraler Zusammensetzung von einer (üblicherweise entfernt vom emittierenden Ende des Leiters angeordneten) Lichtquelle heran. Besonders bevorzugt ist erfindungsgemäß die Verwendung mehrerer, hinsichtlich der Wellenlänge des emittierten Lichts voneinander verschiedener LED's. In einer erfindungsgemäß besonders bevorzugten Ausführungsform werden drei LED's verwendet. Diese können beispielsweise (nicht zwingend und damit nicht beschränkend) blaues, grünes und rotes Licht emittieren.

Der Begriff "Strahlung definierter spektraler Zusammensetzung", wie er in der Beschreibung und in den Patentansprüchen verwendet wird, bedeutet im Zusammenhang mit der vorliegenden Erfindung Strahlung, deren Wellenlängen aus dem kontinuierlichen Spektrum des Lichts, besonders bevorzugt des sichtbaren Lichts, also des Lichts mit Wellenlängen von 400 bis 800 nm, so gewählt werden, daß das eingestrahlte Licht von der Haut-Oberfläche reflektiert werden kann und brauchbare Ergebnisse der Messung des für die Bemessung einer UV-Bestrahlungsdosis relevanten Zustandes der Haut und insbesondere der Messung der Hautfarbe bzw. des Haut-Bräunungszustandes liefert. Es ist im Rahmen der vorliegenden Erfindung möglich, Strahlung mit sich über das gesamte Spektrum erstreckenden Wellenlängen, insbesondere Strahlung des sichtbaren Spektrums des Lichts, zu verwenden, oder auch monochromatische Strahlung zu verwenden. In besonders bevorzugten Ausführungsformen der Erfindung wird Licht spezieller Wellenlängen verwendet, beispielsweise Licht der Wellenlängen im Bereich von etwa 400 bis 450 nm (blau), 470 bis 520 nm (grün) und 700 bis 750 nm (rot) verwendet. Aus Sicht reproduzierbarer Ergebnisse insbesondere der Messungen der Hautfarbe und/oder des Haut-Bräunungszustandes hat sich die Emission von Licht in den drei genannten Wellenlängenbereichen besonders bewährt.

Das Licht definierter spektraler Zusammensetzung wird in einer weiter bevorzugten Ausführungsform geliefert von LED's als Emissionseinrichtung 201. Diese lassen sich erfahrungsgemäß so weit miniaturisieren, daß sie sich einzeln oder zu mehreren in der Einrichtung 200 unterbringen lassen und trotz der geringen Größe das Licht der definierten spektralen Zusammensetzung zuverlässig liefern und auf die Haut einstrahlen.

In einer alternativen Ausführungsform der erfindungsgemäßen Einrichtung 200 wird das später auf die Haut-Oberfläche einzustrahlende Licht definierter spektraler Zusammensetzung an einem von der Einrichtung beabstandeten Ort generiert. Das so generierte Licht - möglich ist eine enge oder auch eine breite spektrale Verteilung der Wellenlängen - wird dann mittels eines oder mehrerer Lichtleiter(s) zu der Emissionseinrichtung 201 geleitet und von dort auf die Haut eingestrahlt.

Die Einrichtung 200 umfasst im Bereich der emittierten Strahlung eine weitere Einrichtung 205, die der Diffusion des emittierten Lichtes zwischen der Emissionseinrichtung 201 und der Hautoberfläche dient und damit dessen Strahlungsrichtung von einem einzigen gerichteten Strahl zu einer Vielzahl diffuser Strahlen ändert.

Als Diffusor 205 wird eine sogenannte "Ulbricht-Kugel" verwendet. Diese hat den Vorteil, daß sie eine diffuse Strahlung erzeugt, die sehr ähnlich derjenigen des natürlichen Sonnenlichts ist. Zur Einstrahlung von Licht für die Messung des für die Bemessung einer UV-Strahlungsdosis relevanten Zustandes der Haut eines Menschen hat diese Lösung den Vorteil, daß eine Meßstrahlung eingestrahlt wird, die derjenigen in einer natürlichen Umgebung weitgehend ähnelt.

Erfindungsgemäß umfasst die Einrichtung 200 weiter einen Sensor 202, der einer Messung der von der Hautoberfläche reflektierten Strahlung oder der Messung der von der Hautoberfläche absorbierten Strahlung (in dem Sinn eingestrahlte Strahlung minus reflektierte Strahlung gleich absorbierte Strahlung) dient, bezogen auf die von der Emissionseinrichtung 201 eingestrahlte Strahlung. Als Sensor 202 können beliebige Sensoren oder Detektoren dienen, wie sie der Fachmann im Bereich der Erfassung von Licht nach Wellenlängen und/oder Intensität kennt. Bevorzugt sind übliche Photodioden. Alternativ dazu könnten auch (gegebenenfalls miniaturisierte) Kameras in dem Fachmann bekannter Form verwendet werden, beispielsweise eine Zeilenkamera oder optische Kamera. Der Sensor 202 kann in besonders bevorzugten Ausführungsformen der Erfindung auch mit zusätzlichen optischen Einrichtungen zusammen verwendet werden, beispielsweise einer oder mehreren Linse(n) 204 zur Fokussierung der reflektierten Strahlung und/oder einem oder mehreren Prisma/Prismen zur Zerlegung der reflektierten Strahlung in nach Wellenlängen unterschiedlicher Strahlung, um auf diesem Weg in der Lage zu sein, auch beim von der Hautoberfläche reflektierten Licht Strahlung einer oder mehrerer definierter Wellenlänge(n) nachzuweisen. Der Sensor 202 mißt die von der Hautoberfläche reflektierte Strahlung in Abhängigkeit von dem für die Bemessung einer UV-Strahlungsdosis relevanten Zustand der Haut des Menschen. Speziell wurde gefunden, dass je nach Farbe der Haut bzw. je nach bereits erfolgter Pigmentierung durch vorangegangene Einstrahlung bräunender UV-Strahlung unterschiedliche Mengen der eingestrahlten Strahlung definierter spektraler Zusammensetzung von der Haut reflektiert werden. Genauer gesagt, reflektiert helle oder noch nicht oder nur schwach vorpigmentierte Haut mehr Strahlung als dunkle oder bereits vorpigmentierte Haut. Die Abhängigkeit der Menge reflektierter Strahlung, bezogen auf eine definierte Menge eingestrahlter Strahlung definierter spektraler Zusammensetzung, ermöglicht eine genaue Abschätzung der Bestrahlungsdosis und/oder Bestrahlungszeit mit überraschend hoher Sicherheit. Insbesondere wird erfindungsgemäß der Tatsache Rechnung getragen, dass verschiedene Körperpartien eines Menschen einen unterschiedlichen, für eine Bemessung der UV-Bestrahlungsdosis relevanten Zustand der Haut aufweisen, insbesondere eine unterschiedliche Hautfarbe und/oder einen unterschiedlichen Haut-Bräunungszustand aufweisen. Dies kann beispielsweise darauf zurückzuführen sein, dass im Alltag die eine Körperpartie (beispielsweise das Gesicht und die Arme) häufiger bräunender UV-Strahlung ausgesetzt sind und damit eine andere Hautfarbe bzw. einen anderen Haut-Bräunungszustand aufweisen als eine andere Körperpartie (beispielsweise der Brustbereich oder der Rücken). Aufgrund (beispielsweise) bereits erfolgter Vorpigmentierung von Gesicht und Armen können diese stärkere UV-Bestrahlungsdosen empfangen und/ oder länger bestrahlt werden als Brust und Rücken. Die Messung des für die Bemessung einer UV-Bestrahlungsdosis relevanten Zustandes der jeweiligen Hautpartien, beispielsweise bevorzugt die Messung der Hautfarbe bzw. des Haut-Bräunungszustands, mittels Emission von Licht definierter spektraler Zusammensetzung und die anschließende Messung der Menge des (einerseits von den vorgebräunten und damit dunkleren Hautpartien des Gesichts und der Arme und andererseits von den noch nicht vorgebräunten und damit helleren Hautpartien der Brust oder des Rückens) reflektierten Lichts liefert ein überraschend gutes Maß für die UV-Bestrahlungsdosis, die auf die auf die Haut eingestrahlt werden kann, oder die mögliche Zeit der Bestrahlung mit UV-Strahlung einer definierten Intensität, ohne dass die Erythemal-Schwelle überschritten wird.

Die von dem Sensor 202 ermittelte Menge reflektierter Strahlung wird über ebenfalls von der Einrichtung 200 umfaßte Einrichtungen 212 an eine Auswert-Einrichtung 203 übertragen. Deren Aufgabe ist es neben der Auswertung der empfangenen Daten, Informationen über die Intensität und Dauer der möglichen Bestrahlung bestimmter Körperpartien mit UV-Strahlung zur Verfügung zu stellen und damit eine Steuerung dahingehend zu initiieren, dass bestimmte Körperpartien weniger und andere stärker bräunender UV-Strahlung ausgesetzt werden. Gemäß der Erfindung kann die Einrichtung 212 auch den Sensor mit der nötigen Energie versorgen.

In einer bevorzugten Ausführungsform der Erfindung umfasst die Einrichtung 200 zusätzlich eine Einrichtung 209 zur Einstellung definierter Abstände zwischen Emissions-Einrichtung 201 und der Haut-Oberfläche einerseits und Sensor-Einrichtung 202 und der Haut-Oberfläche andererseits. Dies wird praktisch mit besonderem Vorteil dadurch verwirklicht, dass die entsprechenden Einrichtungen in fester Anordnung, beispielsweise in einem Messkopf oder Messfühler, verbunden werden. Damit wird eine konkrete Anordnung der jeweiligen Einrichtungen zur Verbesserung der Präzision der Messung und zur Verbesserung der praktischen Handhabung der Einrichtung 200 bei der Messung erreicht.

Die gemäß der vorangehenden Beschreibung aufgebaute und gegebenenfalls weitere Einrichtungen wie Mikroschalter, Kabel, Stecker und/oder bruch- und feuchtigkeitsdichtes Gehäuse umfassende Einrichtung 200 kann zur Messung des für eine Bemessung einer UV-Bestrahlungsdosis relevanten Zustandes der Haut eines Menschen in einer ganzen Anzahl von Anwendungsbereichen eingesetzt werden. So erfolgt eine solche Messung des Zustandes der Haut beispielsweise im Zusammenhang mit der Bestrahlung der Haut mit einen UV-Strahlungsanteil umfassender Strahlung in einem üblichen Bräunungsgerät mit künstlichen UV-Strahlungsquellen wie beispielsweise UV-Strahler-Röhren oder Hochdruck-Brennern. Deren Intensität kann in einer besonders bevorzugten Ausführungsform der Erfindung gemäß dem für die Bestrahlungsdosis relevanten Zustand der Haut des Benutzers eines solchen Bräunungsgeräts gesteuert werden, ganz besonders bevorzugt sogar nach Körperpartien und deren Hautfarbe bzw. Haut-Bräunungszustand getrennt. So werden dunklere und/oder vorpigmentierte Hautpartien wie beispielsweise Gesicht und/oder Arme mit stärkerer Intensität und/oder länger bestrahlt als hellere und/oder weniger (oder gar nicht) vorpigmentierte Hautpartien (Brust, Rücken). An dieser Ausführungsform der Erfindung wird diese beschrieben, ohne jedoch darauf beschränkt zu sein.

Ein weiterer, erfindungsgemäß ebenfalls relevanter und damit bevorzugter Anwendungsbereich der Einrichtung 200 ist die Messung des für eine Bemessung einer UV-Bestrahlungsdosis relevanten Zustandes der Haut eines Menschen im Zusammenhang mit der Bestrahlung der Haut mit einen UV-Strahlungsanteil umfassender Strahlung in der Natur. Spezielle Beispiele hierfür - ohne dass diese als Beschränkung aufgefasst werden sollten - können Vorbereitungen eines Menschen für einen Aufenthalt am Strand oder auf dem Wasser (Segeln, Surfen) oder beim Sport sein, wo natürliche Sonnenstrahlung mit dem natürlichen UV-Anteil zu erwarten ist.

Ein weiterer Anwendungsbereich für die Einrichtung 200, der erfindungsgemäß ebenfalls bevorzugt ist, ist die Messung des für die Bemessung einer UV-Bestrahlungsdosis relevanten Zustandes der Haut eines Menschen im Zusammenhang mit der medizinischen Bestrahlung der Haut mit einen UV-Strahlungsanteil umfassender Strahlung. Bestimmte Krankheiten der Haut wie beispielsweise Akne oder Psoriasis, um nur zwei von ihnen zu nennen, lassen sich durch UV-Bestrahlung der befallenen Haut heilen oder zumindest in ihren Symptomen deutlich lindern. Oft findet eine solche UV-Bestrahlung auch begleitend zu einer Gabe von Medikamenten statt oder kann die Dosis der Medikamentengabe reduzieren oder diese sogar ersetzen. Die gezielte, d. h. hinsichtlich Intensität und Dauer der UV-Bestrahlung auf bestimmte befallene Körperpartien abgestimmte Bestrahlung kann den Heilungsprozeß beschleunigen, insbesondere an den von der Krankheit befallenen Körperpartien, ohne dass das Risiko von Erythemen erhöht wird, die dem kontinuierlichen Heilungsprozeß entgegenstehen.

Ein weiterer, erfindungsgemäß ebenfalls bevorzugter Anwendungsbereich für die Einrichtung 200 ist die Messung des für die Bemessung einer UV-Bestrahlungsdosis relevanten Zustandes der Haut eines Menschen im Zusammenhang mit der kosmetischen Bestrahlung der Haut mit einen UV-Strahlungsanteil umfassender Strahlung. Die Bräunung der Haut aus rein kosmetischen Gründen, zur Verbesserung des Aussehens, zur Schaffung einer gesunderen und strafferen Haut und zur Erhöhung des allgemeinen Wohlbefindens (gegebenenfalls auch zur Vorbereitung eines Aufenthalts in anderen Klimazonen als daheim, in dem stärkere Exposition des Körpers gegenüber UV-Strahlung erwartet wird) treten immer mehr in den Vordergrund. Dadurch wird der Schutz vor überstarker Bestrahlung mit UV-Strahlung immer wichtiger. Die vorliegende Erfindung liefert in besonders vorteilhafter Weise gute Voraussetzungen zur Vermeidung von zu starker Bestrahlung und damit zur Vermeidung von Erythemen, die den Zielen gerade dieser Anwendung im kosmetischen Bereich zuwiderlaufen.

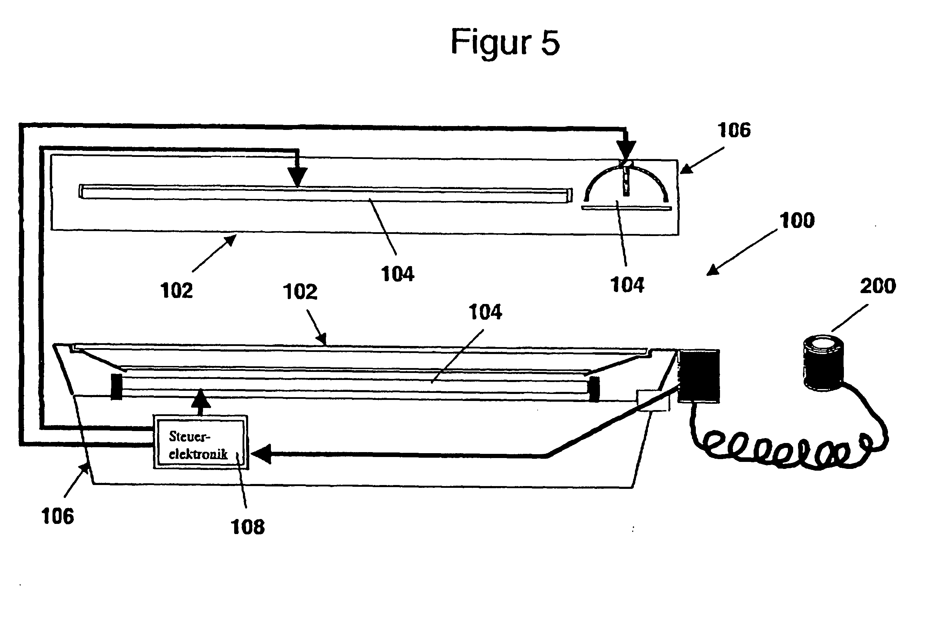

Es wird nun auf die Figur 5 Bezug genommen. Gemäß der vorliegenden Erfindung wird ebenfalls bereitgestellt eine Vorrichtung 100 zur Bestrahlung des Körpers eines Benutzers mit einen UV-Anteil umfassender Strahlung. Die Vorrichtung 100 umfaßt wenigstens eine Einrichtung 106 mit einer dem Benutzer zugewandten, für die Strahlung transparenten Fläche 102 und mehreren, auf der vom Benutzer entfernten Seite der Fläche 102 angeordneten Gruppen von UV-Strahlungsquellen 104, die hinsichtlich Zahl und Anordnung so beschaffen sind, daß sie die einen UV-Anteil umfassende Strahlung im wesentlichen getrennt auf verschiedene Teile des Körpers des Benutzers richten können, und die steuerbar sind durch eine oder mehrere Einrichtung(en) 108 zur Regelung der Leistung der UV-Strahlungsquellen 104, wobei die Regelung der Leistung nach Ergebnissen von Messungen des für die Bemessung der UV-Bestrahlungsdosis relevanten Zustands der Haut des Benutzers an mehreren Stellen seiner Haut-Oberfläche erfolgt.

Die Vorrichtung 100 kann in einer bevorzugten Ausführungsform der Erfindung ein übliches Bräunungsgerät sein, dessen UV-Strahlungsquellen 104 entsprechend den Aufgaben der vorliegenden Erfindung so eingerichtet sind, dass sich einzelne Gruppen von UV-Strahlungsquellen 104 auf verschiedene Teile des Körpers eines Benutzers richten lassen und die jeweiligen, auf verschiedene Teile des Körpers des Benutzers gerichtete UV-Strahlungsquellen 104 hinsichtlich Strahlungsintensität und/oder Bestrahlungsdauer getrennt geregelt werden können. Als UV-Strahlungsquellen 104 kommen in besonders bevorzugten Ausführungsformen der Erfindung beispielsweise UV-Strahler-Röhren oder Hochdruck-Brenner in Frage.

Die Regelung der Leistung der UV-Strahlungsquellen 104 erfolgt erfindungsgemäß entsprechenden Ergebnissen von vor der Bestrahlung durchgeführten Messungen des für die Bemessung der UV-Strahlungsdosis relevanten Zustandes der Haut des Benutzers der Vorrichtung 100, also beispielsweise des Bräunungsgeräts 100. In einer erfindungsgemäß bevorzugten Ausführungsform der Vorrichtung 100 erfolgt die Regelung der Leistung der UV-Strahlungsquellen 104 entsprechend den Ergebnissen von Messungen der Hautfarbe und/oder des aktuellen Haut-Bräunungszustandes des Benutzers. Noch weiter bevorzugt wird erfindungsgemäß eine solche Messung der Hautfarbe und/oder des Haut-Bräunungszustandes des Benutzers mittels einer oder mehrerer Einrichtungen 200 durchgeführt, wie sie oben bereits im Detail unter Bezugnahme auf die Figuren 1 bis 4 beschrieben wurden. Auf der Grundlage der Ergebnisse der mit der/den Einrichtung(en) 200 durchgeführten Messungen, die die Absorption und/oder Reflexion von auf die Haut eingestrahlter Strahlung definierter spektraler Zusammensetzung im Verhältnis zur eingestrahlten Strahlung ermitteln, werden in der Vorrichtung 100 die Einrichtungen 108 angesteuert, die die Leistung der UV-Strahlungsquellen 104 regeln.

In einer weiteren bevorzugten Ausführungsform der Erfindung ist in der Vorrichtung 100 die Zahl der Gruppen von UV-Strahlungsquellen 104 mindestens gleich oder sogar größer als die Zahl der Stellen der Hautoberfläche des Benutzers, an denen Messungen des für eine Bemessung der UV-Bestrahlungsdosis relevanten Zustands der Haut des Benutzers durchgeführt werden, weiter bevorzugt Messungen der Hautfarbe und/oder des Haut-Bräunungszustandes der Haut des Benutzers durchgeführt werden. Mit anderen Worten: Es ist - für eine bestimmte Einrichtung 100 - zu entscheiden, an wie vielen Stellen der Haut-Oberfläche des Benutzers der Einrichtung 100 Messungen beispielsweise der Hautfarbe und/oder des Haut-Bräunungszustandes durchgeführt werden, also wie fein abgestuft die Bräunung des Körpers des Benutzers aufgrund der Konditionierung seiner Haut erfolgen soll. Dementsprechend wird die Zahl der hinsichtlich Intensität oder Bestrahlungsdauer getrennt steuerbaren UV-Strahlungsquellen gleich oder größer als die Zahl der Meßstellen eingerichtet, um eine auf die verschiedenen Körperpartien genau abgestimmte Bräunung der Haut ohne Gefahr des Auftretens von Erythemen aufgrund zu starker Bestrahlung zu erreichen. Beispielhaft können - erfindungsgemäß mit besonderem Vorteil - Gruppen von Einrichtungen 108 zur Regelung der Leistung und/oder Bestrahlungsdauer von UV-Strahlungsquellen 104 entsprechend den Ergebnissen von Messungen des für die UV-Bestrahlungsdosis relevanten Zustands der Haut, insbesondere der Hautfarbe und/oder des Haut-Bräunungszustandes des Benutzers der Vorrichtung 100, an mehreren Stellen seiner Hautoberfläche vorgesehen werden, die gewählt sind aus Haut des Gesichts, Haut des Oberkörpers, Haut des Rückens, Haut des Unterkörpers, Haut des Gesäßes, Haut der Arme und Haut der Beine.

Gemäß einer weiteren bevorzugten Ausführungsform der Erfindung kann die Vorrichtung 100 so gesteuert werden, dass die Einrichtung(en) 200 die Hautfarbe und/oder den Haut-Bräunungszustand des Benutzers vor Beginn der Bestrahlung mit UV-Strahlung misst/messen. Entsprechend dem Meßergebnis wird über die Einrichtung(en) 108 die Intensität der UV-Strahlungsquellen 104 und/oder Dauer der Bestrahlung mit diesen in auf die Hautfarbe und/oder den Haut-Bräunungszustand des Benutzers abgestimmter Weise eingestellt.

In einer weiteren, die Abstimmung verbessernden und damit weiter bevorzugten Ausführungsform der Erfindung misst/messen die Einrichtung(en) 200 die Hautfarbe und/oder den Haut-Bräunungszustand des Benutzers nicht nur vor dem Beginn der Bestrahlung, sondern zusätzlich mindestens einmal während der Bestrahlung, weiter bevorzugt intermittierend oder kontinuierlich während der Bestrahlung. Diese Ausführungsform hat den Vorteil, dass auch während der Bestrahlung Meßergebnisse ermittelt werden, die in Reaktion auf die Bestrahlung entstehen, so dass einer im Laufe der Bestrahlung mit UV-Strahlung entstehender Veränderung der Parameter (Hautfarbe, Haut-Bräunungszustand) Rechnung getragen werden kann.

In einer besonders bevorzugten Ausführungsform umfasst die Vorrichtung 100, die beispielsweise als übliches Bräunungsgerät ausgestaltet sein kann und die vorstehend im Detail beschriebenen Merkmale aufweisen kann,
(a) eine Liege 110 mit einer für die Strahlung transparenten Liegefläche 112 für den Benutzer und Strahlung abgebenden UV-Strahlungsquellen 114;
(b) ein bewegliches Oberteil 120 mit einer für die Strahlung transparenten Schutzfläche 122 und Strahlung abgebenden UV-Strahlungsquellen 124;
(c) wobei das Oberteil 120 im Verhältnis zu der Liege 110 zum Einsteigen eines Benutzers geöffnet und vor Beginn eines Bestrahlungsvorgangs geschlossen werden kann;
(d) vom Benutzer bedienbare Steuergeräte 130 zur Steuerung der Zeit eines Bestrahlungsvorganges; und
(e) eine oder mehrere Einrichtung(en) 108 zur getrennten Regelung der Leistung der UV-Strahlungsquellen 114, 124, wobei die Regelung der Leistung nach Ergebnissen von Messungen der Hautfarbe und/oder des Haut-Bräunungszustandes des Benutzers an mehreren Stellen seiner Hautoberfläche erfolgt.

Die vorstehend beschriebene Vorrichtung 100 in ihrer Ausgestaltung als übliches Bräunungsgerät ist nicht auf die genannte Ausführungsform beschränkt, in der ein Benutzer auf einer Liege liegt und mit UV-Strahlung bestrahlt wird, die von UV-Strahlungsquellen 114, 124 emittiert wird, die unter der Liegefläche 112 und oberhalb der Schutzfläche 122 angeordnet sind. Vielmehr umfaßt die so beschriebene Ausführungsform auch sogenannte Stehbräuner. In diesen sind die UV-Strahlungsquellen vertikal angeordnet, und der Benutzer - selbst stehend - ist von zwei "Hälften" der Vorrichtung 100 umgeben, in denen die UV-Strahlungsquellen 114, 124 um ihn herum angeordnet sind.

Erfindungsgemäß beansprucht wird auch ein Verfahren zur Bestrahlung des Körpers eines Benutzers mit einen UV-Strahlungsanteil umfassender Strahlung aus solche Strahlung emittierenden UV-Strahlungsquellen. Das Verfahren umfaßt den Schritt, daß man den für eine Bemessung einer UV-Bestrahlungsdosis relevanten Zustand der Haut, und insbesondere bevorzugt die Hautfarbe und/oder den Haut-Bräunungszustand, des Benutzers an mehreren Stellen seiner Haut-Oberfläche mißt und entsprechend den Ergebnissen der Messungen die Leistung einzelner Gruppen der UV-Strahler-Röhren regelt, die ihre Strahlung im wesentlichen getrennt auf verschiedene Teile des Körpers des Benutzers richten können.

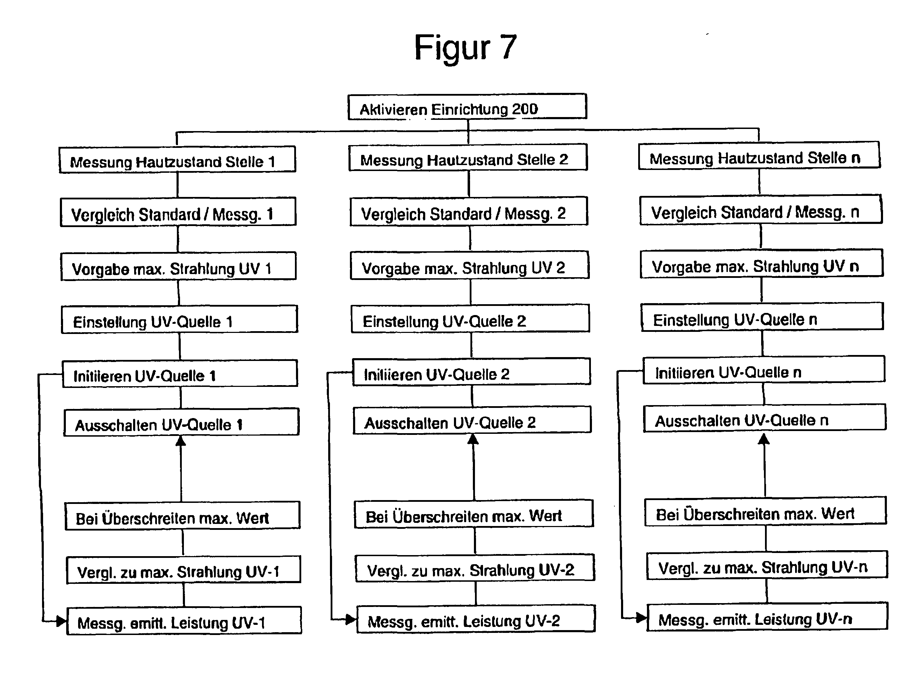

In einem konkreten Fall der Messung des für eine UV-Bestrahlung relevanten Zustands der Haut wird - wie in dem Fließschema in Figur 7 ersichtlich ist - die Einrichtung 200 aktiviert und anschließend die Messung des Hautzustands an mindestens zwei Stellen des Körpers, allgemein an n Stellen des Körpers, des Benutzers durchgeführt, die gewählt sein können beispielsweise aus Haut des Gesichts, Haut des Oberkörpers, Haut des Rückens, Haut des Unterkörpers, Haut des Gesäßes, Haut der Arme und Haut der Beine, ohne dass dies beschränkend gemeint ist. Die Menge der von der Haut des Benutzers reflektierten Strahlung werden vom Sensor 202 erfaßt, und die entsprechenden Daten werden an die Auswerte-Einheit 203 weitergeleitet. Dort werden die Werte der Messung der Strahlungsmenge an der Meßstelle 1, an der Meßstelle 2, ...., an der Meßstelle n mit Standardwerten verglichen, aus denen sich ableiten läßt, wie hoch die maximale Bestrahlungsdosis (oder die maximale Bestrahlungszeit bei einer bestimmten Dosis) für die Stelle sein darf, an der die erste, zweite, ...., n-te Messung durchgeführt wurde. Die UV-Bestrahlungsdosis für die Bestrahlung der ersten, zweiten, ...., n-ten Stelle des Körpers des Benutzers wird entsprechend eingestellt, wobei die Einstellung eine Einstellung der Intensität bei konstanter Zeit der gesamten Bestrahlung, eine Einstellung der Zeit bei konstanter Bestrahlungsdosis oder jede beliebige Kombination von Parametern sein kann, die garantiert, dass die Haut nur so intensiv bestrahlt wird, dass die Erythem-Schwelle nicht erreicht wird. Die UV-Strahlungsquelle, beispielsweise die UV-Strahler-Röhre oder der Hochdruck-Brenner, für die Stelle 1, 2, ...., n wird initiiert und nach Erreichen der Maximalwerte der Dosis, der Zeit und/oder anderer Parameter wieder abgeschaltet.

In einer besonders bevorzugten Ausführungsform der Erfindung kann auch während der Bestrahlung an den Stellen 1, 2 ...., n des Körpers des Benutzers die emittierte Leistung und/oder die seit Initiieren der Strahlungsemission emittierte UV-Strahlung gemessen und mit den vorher als maximal bezeichneten Werten verglichen werden. Sollte ein derartiger Vergleich ein Erreichen oder gar überschreiten der maximalen Werte für eine bestimmte Stelle 1, 2, ...., n ergeben, schaltet das Bräunungsgerät die für die Bestrahlung dieser Stelle oder Körperpartie vorgesehenen UV-Strahlungsquellen einzeln (oder sogar das gesamte Gerät) ab. Auch dies in Figur 7 im unteren Drittel gezeigt.

Die Zahl der Stellen der Haut-Oberfläche des Benutzers, an denen man die relevanten Parameter wie bevorzugt die Hautfarbe und den Haut-Bräunungszustand des Benutzers misst, unterliegt grundsätzlich keinen Beschränkungen. Bevorzugt ist jedoch, dass man die Hautfarbe und/oder den Haut-Bräunungszustand des Benutzers an mindestens zwei Stellen seiner Haut-Oberfläche misst und entsprechend den Ergebnissen der Messungen die Leistung von zwei oder mehr Gruppen der UV-Strahlungsquellen regelt. Besonders bevorzugt ist in dem erfindungsgemäßen Verfahren, die Hautfarbe und/oder den Haut-Bräunungszustand des Benutzers an mindestens zwei Stellen, allgemein an n Stellen, seiner Haut-Oberfläche zu messen, die gewählt sind aus Haut des Gesichts, Haut des Oberkörpers, Haut des Rückens, Haut des Unterkörpers, Haut des Gesäßes, Haut der Arme und Haut der Beine.

Besonders bevorzugt in dem erfindungsgemäßen Verfahren ist, die Messung der Hautfarbe und/oder des Haut-Bräunungszustandes des Benutzers mittels einer oder mehrerer Einrichtung(en) 200 durchzuführen, die die Absorption und/oder Reflexion von auf die Haut eingestrahlter Strahlung definierter spektraler Zusammensetzung im Verhältnis zur eingestrahlten Strahlung messen, und entsprechend dem Ergebnis die Einrichtungen 108 zur Regelung der Leistung der UV-Strahlungsquellen 104 anzusteuern, besonders bevorzugt wie dies in Figur 7 gezeigt ist.

Wie bereits oben im Zusammenhang mit der Vorrichtung 100 ausgeführt, erfolgt beim erfindungsgemäßen Verfahren die Messung des für eine Bemessung der UV-Bestrahlungsdosis relevanten Zustands der Haut des Benutzers vor dem Beginn der Bestrahlung; entsprechend dem Messergebnis wird dann die Bestrahlung gesteuert. In einer anderen, nicht zur Erfindung gehörenden Ausführungsform erfolgt die Messung der Parameter, bevorzugt die Messung der Hautfarbe und/oder des Haut-Bräunungszustands des Benutzers, vor Beginn der Bestrahlung und zusätzlich mindestens einmal während der Bestrahlung, weiter bevorzugt intermittierend oder kontinuierlich während der Bestrahlung. Diese Ausführungsform ist deswegen besonders vorteilhaft, weil sich auch so während der Bestrahlung mit einen UV-Anteil enthaltender Strahlung im Notfall durch Abschalten der entsprechenden Strahlungsquellen oder sogar des gesamten Geräts verhindern läßt, daß die maximalen, gegebenenfalls zur Bildung von Erythemen führenden Parameter der Bestrahlung (Zeit, Intensität usw.) überschritten werden, wenn die Messung der für den Hautzustand relevanten Parameter ergibt, dass die Haut an der Meßstelle für eine derart lange und/oder intensive Bestrahlung nicht geeignet ist.

Das erfindungsgemäße Verfahren findet Anwendung in zahlreichen Bereichen, die erfindungsgemäß nicht beschränkt sind. Bevorzugt sind eine Anwendung des Verfahrens zur kosmetischen Hautbestrahlung und zur medizinischen Hautbestrahlung.

Durch die zumindest vor Beginn der Bestrahlung erfolgende Messung des für die Bemessung einer UV-Bestrahlungsdosis oder der Bestrahlungsdauer relevanten Zustands der Haut eines Benutzers, bevorzugt der Hautfarbe und/oder des Haut-Bräunungszustandes des Benutzers, kann die Bestrahlung mit ultravioletter Strahlung genau auf die individuellen Gegebenheiten unterschiedlich UV-empfindlicher Hautpartien eines Benutzers abgestellt werden. Die Überbestrahlung mit der Folge von Erythem-Bildung ("Sonnenbrand") wird selbst bei empfindlicher heller oder unpigmentierter Haut zuverlässig vermieden. Dies wird erreicht durch ein einfaches, schnell durchzuführendes und apparativ nicht aufwendiges Messverfahren mit der Einrichtung 200 gemäß der vorliegenden Erfindung.

## Patentansprüche

1. Einrichtung (200) zur Messung des für die Bemessung einer UV-Bestrahlungsdosis relevanten Zustands der Haut eines Menschen auf dessen Haut-Oberfläche, umfassend (a) eine Einrichtung (201) zur Emission von Strahlung definierter spektraler Zusammensetzung auf die Haut-Oberfläche; (b) gegebenenfalls eine Einrichtung (211) zum Heranführen der Strahlung definierter Zusammensetzung an die Emissions-Einrichtung (201); (c) einen Sensor (202) zur Messung von von der Hautoberfläche reflektierter oder absorbierter Anteile der von der Emissions-Einrichtung (201) eingestrahlten Strahlung; sowie (d) Einrichtungen (212) zur Versorgung der Einrichtung (201) und/oder des Sensors (202) mit Energie und zur Übertragung von Messdaten an eine Auswert-Einrichtung (203); (e) eine Einrichtung (209) zur Einstellung definierter Abstände zwischen Emissions-Einrichtung (201), Sensor-Einrichtung (202) und der Haut-Oberfläche; sowie (f) eine Einrichtung (205) zum Diffundieren der emittierten Strahlung zwischen der Emissions-Einrichtung (201) und der Hautoberfläche in Form einer Ulbricht-Kugel.

2. Einrichtung (200) nach Anspruch 1, worin die Messung des für die Bemessung einer UV-Bestrahlungsdosis relevanten Zustands der Haut eines Menschen die Messung der Hautfarbe des Menschen und/oder die Messung des Haut-Bräunungszustandes des Menschen ist.

3. Verwendung einer Einrichtung (200) nach einem oder mehreren der Ansprüche 1 oder 2 in einer wenigstens eine Einrichtung (106) mit einer dem Benutzer zugewandten, für die Strahlung transparenten Fläche (102) und mehreren, auf der vom Benutzer entfernten Seite der Fläche (102) angeordneten Gruppen von UV-Strahlungsquellen (104), die hinsichtlich Zahl und Anordnung so beschaffen sind, daß sie die einen UV-Anteil umfassende Strahlung im wesentlichen getrennt auf verschiedene Teile des Körpers des Benutzers richten können, und die steuerbar sind durch eine oder mehrere Einrichtung(en) (108) zur Regelung der Leistung der UV-Strahlungsquellen (104), umfassenden Vorrichtung (100) zur Bestrahlung des Körpers eines Benutzers mit einen UV-Strahlungsanteil umfassender Strahlung zur Regelung der Leistung der UV-Strahlungsquellen nach Ergebnissen von Messungen des für die Bemessung der UV-Bestrahlungsdosis relevanten Zustands der Haut des Benutzers an mehreren Stellen seiner Haut-Oberfläche, worin die Einrichtung(en) (200) die Hautfarbe und/oder den Haut-Bräunungszustand des Benutzers vor dem Beginn der Bestrahlung mißt/messen.

4. Verwendung nach Anspruch 3 zur Regelung der Leistung der UV-Strahlungsquellen nach Ergebnissen von Messungen der Hautfarbe und/oder des Haut-Bräunungszustandes.

5. Verwendung nach Anspruch 3 oder Anspruch 4, worin die Zahl der Gruppen von UV-Strahlungsquellen (104) größer oder gleich der Zahl der Stellen der Haut-Oberfläche des Benutzers ist, an denen Messungen der Hautfarbe und/oder des Haut-Bräunungszustandes des Benutzers erfolgen.

6. Verwendung nach einem der Ansprüche 3 bis 5 in einer mehrere Einrichtungen (108) zur Regelung der Leistung der UV-Strahlungsquellen (104) entsprechend den Ergebnissen von Messungen des Hauttyps und/oder des Haut-Bräunungszustandes des Benutzers an mehreren Stellen seiner Hautoberfläche, die gewählt sind aus Haut des Gesichts, Haut des Oberkörpers, Haut des Rückens, Haut des Unterkörpers, Haut des Gesäßes, Haut der Arme und Haut der Beine, umfassenden Vorrichtung (100) zur Bestrahlung des Körpers eines Benutzers mit einen UV-Strahlungsanteil umfassender Strahlung.

7. Verwendung nach einem der Ansprüche 3 bis 6 zur Messung des Hautfarbe und/oder des Haut-Bräunungszustandes des Benutzers mittels einer oder mehrerer Einrichtung(en) (200), die die Absorption und/oder Reflexion von auf die Haut eingestrahlter Strahlung definierter spektraler Zusammensetzung im Verhältnis zur eingestrahlten Strahlung messen und entsprechend dem Ergebnis die Einrichtungen (108) zur Regelung der Leistung der UV-Strahlungsquellen (104) ansteuern.

8. Verwendung nach einem der Ansprüche 3 bis 7 in einer Vorrichtung (100) zur Bestrahlung des Körpers eines Benutzers mit einen UV-Strahlungsanteil umfassender Strahlung, wobei die Vorrichtung (100) umfaßt:
(a) eine Liege (110) mit einer für die Strahlung transparenten Liegefläche (112) für den Benutzer und Strahlung abgebenden UV-Strahlungsquellen (114);
(b) ein bewegliches Oberteil (120) mit einer für die Strahlung transparenten Schutzfläche (122) und Strahlung abgebenden UV-Strahlungsquellen (124);
(c) wobei das Oberteil (120) im Verhältnis zu der Liege (110) zum Einsteigen eines Benutzers geöffnet und vor Beginn eines Bestrahlungsvorgangs geschlossen werden kann;
(d) vom Benutzer bedienbare Steuergeräte (130) zur Steuerung der Zeit eines Bestrahlungsvorganges; und
(e) eine oder mehrere Einrichtung(en) (108) zur getrennten Regelung der Leistung der UV-Strahlungsquellen (114, 124), wobei die Regelung der Leistung nach Ergebnissen von Messungen der Hautfarbe und/oder des Haut-Bräunungszustandes des Benutzers an mehreren Stellen seiner Hautoberfläche erfolgt.

9. Verwendung nach einem der Ansprüche 3 bis 7, wobei die Vorrichtung (100) umfasst:
(a) eine vertikal angeordnete Hälfte (110) mit einer für die Strahlung transparenten Schutzfläche (112) für den Benutzer und Strahlung abgebenden UV-Strahlungsquellen (114);
(b) eine vertikal angeordnete zweite Hälfte (120) mit einer für die Strahlung transparenten Schutzfläche (122) für den Benutzer und Strahlung abgebenden UV-Strahlungsquellen (124);
(c) wobei eine Hälfte (120) im Verhältnis zu der anderen Hälfte (110) zum Einsteigen eines Benutzers geöffnet und vor Beginn eines Bestrahlungsvorgangs geschlossen werden kann oder beide Hälften zum Einsteigen des Benutzers voneinander weg bewegt und vor Beginn des Bestrahlungsvorgangs durch ein Bewegen in Richtung zueinander geschlossen werden können;
(d) vom Benutzer bedienbare Steuergeräte (130) zur Steuerung der Zeit eines Bestrahlungsvorganges; und
(e) eine oder mehrere Einrichtung(en) (108) zur getrennten Regelung der Leistung der UV-Strahlungsquellen (114, 124), wobei die Regelung der Leistung nach Ergebnissen von Messungen der Hautfarbe und/oder des Haut-Bräunungszustandes des Benutzers an mehreren Stellen seiner Hautoberfläche erfolgt.

10. Verwendung der Einrichtung (200) nach einem der Ansprüche 1 oder 2 zur Messung des für eine Bemessung einer UV-Bestrahlungsdosis relevanten Zustandes der Haut eines Menschen vor der Bestrahlung der Haut mit einen UV-Strahlungsanteil umfassender Strahlung in einem Bräunungsgerät mit künstlichen UV-Strahlungsquellen.

11. Verwendung der Einrichtung (200) nach einem der Ansprüche oder 2 zur Messung des für eine Bemessung einer UV-Bestrahlungsdosis relevanten Zustandes der Haut eines Menschen vor der Bestrahlung der Haut mit einen UV-Strahlungsanteil umfassender Strahlung in der Natur.

12. Verwendung der Einrichtung (200) nach einem der Ansprüche oder 2 zur Messung des für eine Bemessung einer UV-Bestrahlungsdosis relevanten Zustandes der Haut eines Menschen vor der medizinischen Bestrahlung der Haut mit einen UV-Strahlungsanteil umfassender Strahlung.

13. Verwendung der Einrichtung (200) nach einem der Ansprüche oder 2 zur Messung des für eine Bemessung einer UV-Bestrahlungsdosis relevanten Zustandes der Haut eines Menschen vor der kosmetischen Bestrahlung der Haut mit einen UV-Strahlungsanteil umfassender Strahlung.

14. Verwendung nach einem der Ansprüche 10 bis 13 zur Messung der Hautfarbe und/oder des Haut-Bräunungszustandes der Haut eines Menschen.

15. Verfahren zur Ermittlung des für eine Bemessung einer UV-Bestrahlungsdosis relevanten Zustand der Haut des Benutzers eines Bräunungsgeräts, in dem man die Absorption und/oder Reflexion von auf die Haut eingestrahlter Strahlung definierter spektraler Zusammensetzung im Verhältnis zur eingestrahlten Strahlung an mehreren Stellen seiner Haut-Oberfläche vor dem Beginn der UV-Bestrahlung mittels einer oder mehrerer Einrichtung(en) (200) gemäß den Ansprüchen 1 oder 2 mißt und entsprechend den Ergebnissen der Messungen die Leistung einzelner Gruppen der UV-Strahler-Röhren des Bräunungsgeräts regelt, die ihre Strahlung im wesentlichen getrennt auf verschiedene Teile des Körpers des Benutzers richten können.

16. Verfahren nach Anspruch 15, umfassend den Schritt, daß man die Hautfarbe und/oder den Haut-Bräunungszustand des Benutzers vor dem Beginn der UV-Bestrahlung an mindestens zwei Stellen seiner Haut-Oberfläche mißt und entsprechend den Ergebnissen der Messungen die Leistung von zwei oder mehr als zwei Gruppen der UV-Strahlungsquellen regelt.

17. Verfahren nach Anspruch 15 oder Anspruch 16, umfassend den Schritt, daß man die Hautfarbe und/oder den Haut-Bräunungszustand des Benutzers vor dem Beginn der Bestrahlung an mindestens zwei Stellen seiner Haut-Oberfläche mißt, die gewählt sind aus Haut des Gesichts, Haut des Oberkörpers, Haut des Rückens, Haut des Unterkörpers, Haut des Gesäßes, Haut der Arme und Haut der Beine.

18. Verfahren nach einem oder mehreren der Ansprüche 15 bis 17, umfassend den Schritt, daß man die Messung der Hautfarbe und/oder des Haut-Bräunungszustandes des Benutzers vor dem Beginn der UV-Bestrahlung mittels einer oder mehrerer Einrichtung(en) (200) durchführt, die die Absorption und/oder Reflexion von auf die Haut eingestrahlter Strahlung definierter spektraler Zusammensetzung im Verhältnis zur eingestrahlten Strahlung messen, und daß man entsprechend dem Ergebnis die Einrichtungen (108) zur Regelung der Leistung der UV-Strahlungsquellen (104) ansteuert.

## Claims

1. A device (200) for a measurement of the status, as far as relevant for determining the UV irradiation dose, of the skin of a person on his/her skin, comprising (a) a means (201) for emitting radiation having a defined spectral composition onto the skin; (b) optionally a means (211) for advancing the radiation of a defined composition to the emission means (201); (c) a sensor (202) for measuring the portion of the radiation irradiated by the emission means (201) which is reflected or absorbed by the skin; and (d) means (212) for providing energy to the means (201) and/or to the sensor (202) and for transmitting measured data to a data analyzing means (203); (e) a means (209) for adjusting defined distances between the emission means (201), the sensor means (202) and the skin; and (f) a means (205) for diffusing the emitted radiation between the emission means (201) and the skin in the form of an Ulbricht sphere.

2. The device (200) according to claim 1, wherein the measurement of the status, as far as relevant for determining the UV irradiation dose, of a person's skin is a measurement of the skin color of said person and/or a measurement of the skin tan status of said person.

3. A use of the device (200) according to one or more than one of claims 1 or 2 in an apparatus (100) for irradiating radiation comprising a UV radiation portion onto the body of a user, said apparatus comprising at least one means (106) having a surface (102) facing the user and being transparent for the radiation and having several groups of UV radiation sources (104) arranged on that side of the surface (102) which is turned away from the user, which UV radiation sources (104) are designed, with respect to their number and position, in such a way that they can direct radiation comprising a UV radiation portion substantially separately onto different parts of the user's body, and are controllable by one or more means (108) for controlling the power of the UV radiation sources (104), for controlling the power of UV radiation sources in accordance with the results of the measurements of the status, as far as relevant for determining the UV irradiation dose, of the user's skin at several points of his/her skin, wherein the means (200) measure(s) the skin color and/or the skin tan status of the user before beginning the irradiation.

4. The use according to claim 3 for controlling the power of the UV radiation sources in accordance with the measurements of the skin color and/or of the skin tan status.

5. The use according to claim 3 or claim 4, wherein the number of groups of UV radiation sources (104) is larger than or equal to the number of points of the user's skin where measurements of the skin color and/or the skin tan status of the user are effected.

6. The use according to any of the claims 3 to 5 in an apparatus (100) for irradiating radiation comprising a UV radiation portion onto the body of a user, said apparatus comprising several means (108) for controlling the power of the UV radiation sources (104) in accordance with the results of the measurements of the user's skin type and/or the user's skin tan status at several points of the skin selected from skin of the face, skin of the chest, skin of the back, skin of the belly, skin of the bottom, skin of the arms and skin of the legs.

7. The use according to any of the claims 3 to 6 for the measurement of the skin color and/or of the skin tan status of the user by means of one or more means (200) measuring the absorption and/or the reflection of radiation having a defined spectral composition and irradiated onto the skin in relation to the irradiated radiation and addressing the means (108) controlling the power of the UV radiation sources (104) in accordance with result of said measurement.

8. The use according to any of the claims 3 to 7 in an apparatus (100) for irradiating, onto the body of a user, radiation comprising UV radiation portion, said apparatus (100) comprising
(a) a bed (110) with a surface (112) for the user transparent for the radiation, and UV radiation sources (114) emitting radiation;
(b) a movable upper part (120) having a protection surface (122) transparent for the radiation, and UV radiation sources (124) emitting radiation;
(c) wherein the upper part (120), in relation to the bed (110) may be opened for allowing a user to get in and may be closed before starting the irradiation process;
(d) controlling means (130) for controlling the time of an irradiation process which may be used by the user; and
(e) one or more device(s) (108) for separately controlling the power of the UV radiation sources (114, 124), wherein the control of the power is effected in accordance with the results of measurements of the skin color and/or of the skin tan status of the user at several points of his skin.

9. The use according to any of the claims 3 to 7, said apparatus (100) comprising
(a) a first half (110) arranged vertically and having a protection surface (112) for the user transparent for the radiation, and UV radiation sources (114) emitting radiation;
(b) a second half (120) arranged vertically and having a transparent protection surface (122) for the user transparent for the radiation, and UV radiation sources (124) emitting radiation;
(c) whereby the second half (120) may be opened with respect to the first half (110) for allowing a user to get in and may be closed before initiating the irradiation process, or both halves may be moved apart from each other for allowing a user to get in and may be closed by moving them towards each other before initiating the irradiation process;
(d) control apparatus (130) for controlling the time of the irradiation process, which may be operated by the user; and
(e) one or more means (108) for separately controlling the power of the UV radiation sources (114, 124), wherein a control of the power is effected in accordance with the results of the measurements of the skin color and/or the skin tan status of the user at several points of the user's skin.

10. The use of the device (200) according to any of the claims 1 or 2 for measuring the status, as far as relevant for determining the UV irradiation dose, of a person's skin in connection with the irradiation, onto the skin, of radiation comprising a UV radiation portion in a tanning apparatus having artificial UV radiation sources.

11. The use of the device (200) according to any of the claims 1 or 2 for measuring the status, as far as relevant for determining the UV irradiation dose, of a person's skin in connection with the irradiation, onto the skin, of radiation comprising a UV radiation portion in nature.

12. The use of the device (200) according to any of the claims 1 or 2 for measuring the status, as far as relevant for determining the UV irradiation dose, of a person's skin in connection with the medical irradiation, onto the skin, of radiation comprising a UV radiation portion.

13. The use of the device (200) according to any of the claims 1 or 2 for measuring the status, as far as relevant for determining the UV irradiation dose, of a person's skin in connection with the cosmetic irradiation, onto the skin, of radiation comprising a UV radiation portion.

14. The use according to any of the claims 10 to 13 for measuring the color of the skin or the skin tan status of a person's skin.

15. A process for determining the status, as far as relevant for determining the UV irradiation dose, of the skin of a user of a tanning device, by measuring, at several points of the user's skin, the absorption and/or reflection of radiation irradiated onto the skin and having a defined spectral composition in relation to the irradiated radiation before beginning the UV irradiation by means of one or several devices (200) according to claims 1 or 2 and controlling the power of single groups of UV radiation tubes of the tanning device in accordance with the results of said measurements, which tubes are capable of directing their radiation substantially separately onto different parts of the user's body.

16. The process according to claim 15, comprising the steps of measuring the user's skin color and/or skin tan status at at least two points of the skin before beginning the UV irradiation and of controlling the power of two or more than two groups of UV radiation sources in accordance with the results of said measurements.

17. The process according to claim 15 or claim 16, comprising the steps of measuring the user's skin color and/or skin tan status at at least two points of the skin before beginning the UV irradiation, which points are selected from skin of the face, skin of the chest, skin of the back, skin of the belly, skin of the bottom, skin of the arms and skin of the legs.

18. The process according to one or several of the claims 15 to 17, comprising the steps of conducting the measurement of the user's skin color and/or skin tan status before beginning the UV irradiation by means of one or several device(s) (200) measuring the absorption and/or reflection of radiation irradiated onto the skin and having a defined spectral composition in relation to the irradiated radiation and addressing the means (108) controlling the power of the UV radiation sources (104) in accordance with result of said measurement.

## Revendications

1. Dispositif (200) pour mesurer sur la surface de la peau d'un être humain l'état de la peau dudit être humain pertinent pour le calcul d'une dose de rayonnement UV, comprenant (a) un dispositif (201) pour émettre sur la surface de la peau un rayonnement de composition spectrale définie, (b) le cas échéant, un dispositif (211) pour amener le rayonnement de composition définie au dispositif d'émission (201), (c) un capteur (202) pour mesurer des fractions du rayonnement incident du dispositif d'émission (201) qui sont réfléchies ou absorbées par la surface de la peau, et (d) des dispositifs (212) pour alimenter le dispositif (201) et/ou le capteur (202) en énergie et pour transmettre des données de mesure à un dispositif d'évaluation (203), (e) un dispositif (209) pour le réglage de distances définies entre le dispositif d'émission (201), le dispositif de détection (202) et la surface de la peau, ainsi que (f) un dispositif (205) pour diffuser le rayonnement émis entre le dispositif d'émission (201) et la surface de la peau sous la forme d'une sphère d'Ulbricht.

2. Dispositif (200) selon la revendication 1, dans lequel la mesure de l'état de la peau d'un être humain pertinent pour le calcul d'une dose de rayonnement UV est la mesure de la couleur de la peau de l'être humain et/ou la mesure de l'état de bronzage de la peau de l'être humain.

3. Utilisation d'un dispositif (200) selon l'une ou plusieurs des revendications 1 ou 2 dans un dispositif (100) comportant au moins un dispositif (106) avec une surface (102) transparente pour le rayonnement et tournée vers l'utilisateur et plusieurs groupes de sources de rayonnement UV (104) disposés sur le côté de la surface (102) qui est éloigné de l'utilisateur, lesquels sont tels, au regard de leur nombre et de leur agencement, qu'ils peuvent diriger le rayonnement comprenant une fraction UV de manière sensiblement distincte sur différentes parties du corps de l'utilisateur et lesquels peuvent être commandés par un ou plusieurs dispositifs (108) pour régler la puissance des sources de rayonnement (UV) (104), ledit dispositif (100) étant destiné à exposer le corps d'un utilisateur à un rayonnement comprenant une fraction de rayonnement UV afin de régler la puissance des sources de rayonnement UV en fonction de résultats de mesures de l'état de la peau de l'utilisateur pertinent pour le calcul de la dose de rayonnement UV effectuées en plusieurs points de la surface de sa peau, le ou les dispositifs (200) mesurant la couleur de la peau et/ou l'état de bronzage de la peau de l'utilisateur avant le début de l'exposition au rayonnement.

4. Utilisation selon la revendication 3 pour le réglage de la puissance des sources de rayonnement UV en fonction de mesures de la couleur de la peau et/ou de l'état de bronzage de la peau.

5. Utilisation selon la revendication 3 ou la revendication 4, dans laquelle le nombre de groupes de sources de rayonnement UV (104) est supérieur ou égal au nombre de points de la surface de la peau de l'utilisateur auxquels sont effectuées des mesures de la couleur de la peau et/ou de l'état de bronzage de la peau de l'utilisateur.

6. Utilisation selon l'une des revendications 3 à 5 dans un dispositif (100) qui est destiné à exposer le corps d'un utilisateur à un rayonnement comprenant une fraction de rayonnement UV et qui comporte plusieurs dispositifs (108) pour le réglage de la puissance des sources de rayonnement UV (104) en fonction des résultats de mesures du type de peau et/ou de l'état de bronzage de la peau de l'utilisateur effectuées en plusieurs points de la surface de sa peau choisis sur la peau du visage, la peau de la partie supérieure de corps, la peau du dos, la peau de la partie inférieure du corps, la peau du derrière, la peau des bras et la peau des jambes.

7. Utilisation selon l'une des revendications 3 à 6 pour mesurer la couleur de la peau et/ou l'état de bronzage de la peau de l'utilisateur au moyen d'un ou de plusieurs dispositifs (200) qui mesurent l'absorption et/ou la réflexion d'un rayonnement de composition spectrale définie frappant la peau en relation avec le rayonnement incident et qui commandent les dispositifs (108) de réglage de la puissance des sources de rayonnement UV (104) en fonction du résultat.

8. Utilisation selon l'une des revendications 3 à 7 dans un dispositif (100) destiné à exposer le corps d'un utilisateur à un rayonnement comprenant une fraction de rayonnement UV, le dispositif (100) comprenant :
(a) un banc (110) avec une surface (112) transparente pour le rayonnement pour l'utilisateur et des sources de rayonnement UV (114) qui émettent un rayonnement ;
(b) une partie supérieure mobile (120) avec une surface de protection (122) transparente pour le rayonnement et des sources de rayonnement UV (124) qui émettent un rayonnement ;
(c) la partie supérieure (120) pouvant être ouverte par rapport au banc (110) pour permettre à un utilisateur d'entrer et pouvant être refermée avant le début d'une exposition au rayonnement ;
(d) des appareils de commande (130) qui peuvent être manipulés par l'utilisateur pour commander la durée d'une exposition au rayonnement ; et
(e) un ou plusieurs dispositifs (108) pour le réglage séparé de la puissance des sources de rayonnement UV (114, 124), le réglage de la puissance étant effectué en fonction de résultats de mesures de la couleur de la peau et/ou de l'état de bronzage de la peau de l'utilisateur effectuées en plusieurs points de la surface de sa peau.

9. Utilisation selon l'une des revendications 3 à 7, le dispositif (100) comprenant :
(a) une moitié verticale (110) avec une surface de protection (112) transparente pour le rayonnement pour l'utilisateur et des sources de rayonnement UV (114) qui émettent un rayonnement ;
(b) une deuxième moitié verticale (120) avec une surface de protection (122) transparente pour le rayonnement pour l'utilisateur et des sources de rayonnement UV (124) qui émettent un rayonnement ;
(c) une moitié (120) pouvant être ouverte par rapport à l'autre moitié (110) pour permettre à un utilisateur d'entrer et pouvant être refermée avant le début d'une exposition au rayonnement ou les deux moitiés pouvant être écartées l'une de l'autre pour permettre à l'utilisateur d'entrer et pouvant être refermées par un mouvement de rapprochement l'une vers l'autre avant le début d'une exposition au rayonnement ;
(d) des appareils de commande (130) qui peuvent être manipulés par l'utilisateur pour commander la durée d'une exposition au rayonnement ; et
(e) un ou plusieurs dispositifs (108) pour le réglage séparé de la puissance des sources de rayonnement UV (114, 124), le réglage de la puissance étant effectué en fonction de résultats de mesures de la couleur de la peau et/ou de l'état de bronzage de la peau de l'utilisateur effectuées en plusieurs points de la surface de sa peau.

10. Utilisation du dispositif (200) selon l'une des revendications 1 ou 2 pour mesurer l'état de la peau d'un être humain pertinent pour calculer une dose de rayonnement UV avant l'exposition de la peau à un rayonnement comprenant une fraction de rayonnement UV dans un appareil de bronzage avec des sources artificielles de rayonnement UV.

11. Utilisation du dispositif (200) selon l'une des revendications 1 ou 2 pour mesurer l'état de la peau d'un être humain pertinent pour calculer une dose de rayonnement UV avant l'exposition de la peau à un rayonnement naturel comprenant une fraction de rayonnement UV.

12. Utilisation du dispositif (200) selon l'une des revendications 1 ou 2 pour mesurer l'état de la peau d'un être humain pertinent pour calculer une dose de rayonnement UV avant l'exposition de la peau à un rayonnement comprenant une fraction de rayonnement UV à des fins médicales.

13. Utilisation du dispositif (200) selon l'une des revendications 1 ou 2 pour mesurer l'état de la peau d'un être humain pertinent pour calculer une dose de rayonnement UV avant l'exposition de la peau à un rayonnement comprenant une fraction de rayonnement UV à des fins cosmétiques.

14. Utilisation selon l'une des revendications 10 à 13 pour mesurer la couleur de la peau et/ou l'état de bronzage de la peau d'un être humain.

15. Procédé pour déterminer l'état de la peau de l'utilisateur d'un appareil de bronzage pertinent pour calculer une dose de rayonnement UV, dans lequel on mesure l'absorption et/ou la réflexion d'un rayonnement de composition spectrale déterminée frappant la peau en relation avec le rayonnement incident en plusieurs points de la surface de sa peau avant le début de l'exposition au rayonnement UV au moyen d'un ou de plusieurs dispositifs (200) selon les revendications 1 ou 2 et on règle, en fonction des résultats des mesures, la puissance de groupes distincts de tubes d'irradiation UV de l'appareil de bronzage qui peuvent diriger leur rayonnement de manière sensiblement distincte sur différentes parties du corps de l'utilisateur.

16. Procédé selon la revendication 15, comprenant l'étape dans laquelle on mesure la couleur de la peau et/ou l'état de bronzage de la peau de l'utilisateur avant le début de l'exposition au rayonnement UV en au moins deux points de la surface de sa peau et on règle la puissance de deux ou de plus de deux groupes de sources de rayonnement UV en fonction des résultats des mesures.

17. Procédé selon la revendication 15 ou la revendication 16, comprenant l'étape dans laquelle on mesure la couleur de la peau et/ou l'état de bronzage de la peau de l'utilisateur avant le début de l'exposition au rayonnement en au moins deux points de la surface de sa peau choisis sur la peau du visage, la peau de la partie supérieure de corps, la peau du dos, la peau de la partie inférieure du corps, la peau du derrière, la peau des bras et la peau des jambes.

18. Procédé selon l'une ou plusieurs des revendications 15 à 17, comprenant l'étape dans laquelle on effectue la mesure de la couleur de la peau et/ou de l'état de bronzage de la peau de l'utilisateur avant le début de l'exposition au rayonnement UV au moyen d'un ou de plusieurs dispositifs (200) qui mesurent l'absorption et/ou la réflexion d'un rayonnement de composition spectrale définie frappant la peau en relation avec le rayonnement incident et on commande les dispositifs (108) de réglage de la puissance des sources de rayonnement UV (104) en fonction du résultat.
